# EUROPEAN PATENT SPECIFICATION

(11) **EP 1 733 732 B1**
(45) Date of publication and mention of the grant of the patent: **10.10.2012**
(21) Application number: 05721449.6
(22) Date of filing: 25.03.2005
(51) Int. Cl.: A61K 31/785, A61K 31/74, A61K 31/787, A61K 45/00, A61P 3/06, A61P 3/10, A61P 5/50, A61P 9/00, A61P 9/10, A61P 9/12, A61P 13/12, A61P 19/06

(54) **INSULIN RESISTANCE IMPROVING AGENT**
MITTEL ZUR VERBESSERUNG DER INSULINRESISTENZ
AGENT AMELIORANT LA RESISTANCE A L INSULINE

(30) Priority: 26.03.2004 JP 2004091508
(43) Date of publication of application: 20.12.2006
(60) Divisional application: 11002551.7
(73) Proprietor: Mitsubishi Tanabe Pharma Corporation, Osaka-shi Osaka 541-8505 (JP)
(72) Inventor: SUZUKI, Kazuo, c/o MITSUBISHI PHARMA CORPORATION, Tokyo 1038405 (JP); NAKAJIMA, Shigekazu, c/o MITSUBISHI PHARMA CORP., Tokyo 1038405 (JP); SUGIMOTO Kanami, c/o MITSUBISHI PHARMA CORPORATION, Tokyo 1038405 (JP)
(74) Representative: Merkle, Gebhard
(86) International application number: PCT/JP2005/005472
(87) International publication number: WO 2005/092349

(56) References cited:
- WO-A-02/085377
- WO-A1-03/011308
- JP-A- 2002 537 390
- JP-A- 2003 055 235
- US-A- 5 298 497
- US-A1- 2002 169 146
- US-A1- 2003 175 237
- GARG A. ET AL: 'Cholestyramine Therapy for Dyslipidemia in Non-Insulin-dependent Diabetes Mellitus.' ANN.INTERN.MED. vol. 121, 1994, pages 416 - 422, XP002989475
- SKYLER JAY S ET AL: "Hypertension in patients with diabetes mellitus", AMERICAN JOURNAL OF HYPERTENSION, vol. 8, no. 12 PART 2, 1995, pages 100S-105S, ISSN: 0895-7061

## Description

### Technical Field

The present invention relates to an insulin resistance-improving agent comprising a pharmaceutically acceptable anion exchange resin as an active ingredient.

### Background Art

As for anion exchange resins known as hypocholesterolemic agents, including colestimide (trade name: Cholebine, Mitsubishi Pharma Corporation) as a typical example, reports were made on hypoglycemic action after administration over a certain period of time (Non-patent document 1) and an effect on the circadian variation of blood glucose levels in hypercholesterolemia patients also suffering from type 2 diabetes (Patent document 1). However, modes of mechanisms thereof remain unknown. For example, cholestyramine resin is reported to have a hypoglycemic action (Non-patent document 2), and the mechanism is considered to be not associated with improvement of insulin resistance, but with inhibition of gastrointestinal motility via cholecystokinin (CCK) and promotion of insulin secretion.

WO 03/011308 A1 discloses drugs for ameliorating postcibal hyperglycemia, drugs for inhibiting an increase in blood glucose level and compositions for preventing or treating diabetics containing a pharmacologically acceptable anion exchange resin typified by colestimide.

Garg A. et al.: "Cholestyramine Therapy for Dyslipidemia in Non-Insulin-dependent Diabetes Mellitus.", Ann. Intern. Med., Vol. 121, 1994, Pages 416-422, XP002989475 discloses the clinical efficacy and tolerability of cholestyramine therapy in patients with dyslipidemia and non-insulin-dependent diabetes mellitus.

US-A-5 298 497 discloses a method for preventing or reducing the risk of hypertension in normotensive patients having insulin resistance by administering a cholesterol lowering drug.

JP 2002 537390 A discloses hypolipidemic, antihyperglycemic, antiobesity and hypocholesterolemic compounds and derivatives thereof.

JP 2003 055235 A discloses a cholesterol-lowering agent, comprising a polymer composed of (a) a vinyl monomer in which the degree of swelling in water is ≥ 10 times and containing a quaternary ammonium salt and (b) a crosslinkable monomer.

WO 02/085377 A discloses a method for treating hyperglycemia and/or reducing serum glucose levels in a patient that includes administering to the patient a therapeutically effective amount of an amine polymer.

US 2003/175237 A1 discloses a method of reducing levels of fasting plasma glucose and/or hemoglobin A 1C in patients by the administration of an aliphatic amine polymer, such as colesevelam HCl.

US 2002/169146 A1 discloses pharmaceutically acceptable salts of compounds of the general formula (I).

Insulin preparations, sulfonylurea agents, and rapid-acting insulin secretagogues, which exhibit hypoglycemic actions, basically have no insulin resistance-improving action. α-Glucosidase inhibitors that delay glucose absorption have only a slight indirect insulin resistance-improving action. Therefore, agents having a hypoglycemic action are not generally considered to have an insulin resistance-improving action. Since these agents are non-absorbable, it is believed that an artisan would not have been able to relate the agents to improvement of resistance in the peripheral muscles and the like or improvement in the liver.

At present, a class of glitazone antidiabetic drugs are known as typical examples of insulin resistance-improving agents. From a viewpoint of mode of action, the glitazone drugs have an adipocyte differentiation-inducing action, which action improves glucose uptake ability of adipocytes to lower the blood glucose level.
However, as an adverse reaction, the drugs increases adipose tissues (i.e., aggravation of obesity). In addition, since retention of humor (edema) was reported, these drugs are requested to be administered carefully to patients with decreased cardiac function frequently observed in diabetes patients. Further, since they have a risk of hepatotoxicity, liver function tests should be frequently performed.

Therefore, a novel medicament that solves the aforementioned problems, specifically, a non-absorbable medicament that improves insulin resistance without the increase of body weight (fat) and can be used without the risk of inducing edema or hepatotoxicity has been desired.

Further, to the best knowledge of the inventors of the present invention, no report has been made so far that pharmaceutically acceptable anion exchange resins exhibit an insulin resistance-improving action.
Patent document 1: WO03/011398
Non-patent document 1: Rinsho Iyaku (Journal of Clinical Therapeutics & Medicine), Vol. 12, No. 8, June 1996, p.1641
Non-patent document 2: Ann. Intern. Med., 1994; 121: pp.416-422

### Disclosure of the Invention

### Object to be Achieved by the Invention

Accordingly, an object of the present invention is to provide an insulin resistance-improving agent comprising a pharmaceutically acceptable anion exchange resin as an active ingredient.

### Means for Achieving the Object

The inventors of the present invention conducted various researches to achieve the foregoing object. As a result, they found that colestimide and colesevelam hydrochloride known as hypocholesterolemic agents and sevelamer hydrochloride known as a hyperphosphatemia curing agent exhibited an apparent insulin resistance-improving action and thus achieved the present invention.

The gists of the present invention are as follows:
1. An agent comprising a pharmaceutically acceptable anion exchange resin as an active ingredient for the improvement of insulin resistance.
2. The agent according to claim 1, wherein the pharmaceutically acceptable anion exchange resin has a bile acid-adsorbing ability.
3. The agent according to claim 1 or 2, wherein the pharmaceutically acceptable anion exchange resin is selected from the grouop consisting of colestimide, cholestyramine resin, colestipol, sevelamer hydrochloride, and colesevelam hydrochloride.
4. The agent according to claim 1 or 2, wherein the pharmaceutically acceptable anion exchange resin is an anion exchange resin synthesized by a polymerization reaction of an epichlorohydrin derivative and an amine of which typical example includes an imidazole derivative.
5. The agent according to any one of claims 1 to 4, wherein the pharmaceutically acceptable anion exchange resin is colestimide.
6. The agent according to any one of claims 1 to 5, with which an oral hypoglycemic agent is used simultaneously, separately, or successively.
7. The agent according to claim 6, wherein the oral hypoglycemic agent is selected from the group consisting of α-glucosidase inhibitors, biguanides, insulin sensitivity improving agents, sulfonylurea agents, rapid-acting insulin secretagogues, pharmaceutical preparations comprising GLP-1 or derivatives thereof, and DPP-IV inhibitors.
8. An agent for the prophylactic, improving and/or therapeutic treatment of a disease or symptom resulting from insulin resistance, which comprises a pharmaceutically acceptable anion exchange resin as an active ingredient, wherein the disease or symptom resulting from insulin resistance is selected from the group consisting of hyperinsulinism and fatty liver.
9. The agent according to claim 8, wherein the disease or symptom resulting from insulin resistance is hyperinsulinism.
10. The agent according to claim 8, wherein the disease or symptom resulting from insulin resistance is fatty liver.
11. The agent according to any one of claims 8 to 10, wherein the agent is one for prophylactic treatment of the disease or symptom resulting from insulin resistance.
12. The agent according to any one of claims 8 to 10, wherein the agent is one for improving treatment of the disease or symptom resulting from insulin resistance.
13. The agent according to any one of claims 8 to 10, wherein the agent is one for therapeutic treatment of the disease or symptom resulting from insulin resistance.
14. The agent according to any one of claims 8 to 13, wherein the pharmaceutically acceptable anion exchange resin has a bile acid adsorbing ability.
15. The agent according to any one of claims 8 to 14, wherein the pharmaceutically acceptable anion exchange resin is selected from the group consisting of colestimide, cholestyramine resin, colestipol, sevelamer hydrochloride, and colesevelam hydrochloride.
16. The agent according to any one of claims 8 to 15, wherein the pharmaceutically acceptable anion exchange resin is an anion exchange resin synthesized by a polymerization reaction of an epichlorohydrin derivative and an amine of which typical example includes an imidazole derivative.
17. The agent according to any one of claims 8 to 16, wherein the pharmaceutically acceptable anion exchange resin is colestimide.
18. The agent according to any one of claims 8 to 17, with which an oral hypoglycemic agent is used simultaneously, separately, or successively.
19. The agent according to claim 18, wherein the oral hypoglycemic agent is selected from the group consisting of -glucosidase inhibitors, biguanides, insulin sensitivity improving agents, sulfonylurea agents, rapid-acting insulin secretagogues, pharmaceutical preparations comprising GLP-1 or derivatives thereof, and DPP-IV inhibitors.

### Effect of the Invention

According to the present invention, a medicament can be provided which exhibits an insulin resistance-improving action regardless of a volume of meal or glucose absorption from the gastrointestinal tract.

### Brief Description of the Drawings

[Fig. 1] Drawing which depicts changes in body weight in Example 1.
[Fig. 2] Drawing which depicts changes in feed intake in Example 1.
[Fig. 3] Drawings which depicts comparison between the control group and the colestimide prophylactic treatment group for average feed intake in the 1st to 12th weeks of the test period in Example 1 (Fig. 3-1) and comparison across the control group, the colestimide prophylactic treatment group, and the colestimide therapeutic treatment group for average feed intake in the 12th to 20th weeks of the test period (Fig. 3-2).
[Fig. 4] Drawing which depicts changes in plasma blood glucose levels in Example 1.
[Fig. 5] Drawing which depicts changes in plasma insulin levels in Example 1.
[Fig. 6] Drawing which depicts the insulin sensitivity indices (insulin resistance-improving action) in Example 1.
[Fig. 7] Drawing which depicts glucose uptake into peripheral tissues associated with the insulin resistance-improving action in Example 1.
[Fig. 8] Drawings which depicts fasting blood glucose levels in the control group and the colestimide group (Fig. 8-1), fasting insulin levels in the colestimide group (Fig. 8-2) and blood glucose level AUCs (0 to 120 min) in the colestimide group (Fig. 8-3) in Example 2.
[Fig. 9] Drawings which depicts fasting blood glucose levels in the control group and the colesevelam hydrochloride group (Fig. 9-1), fasting insulin levels in the colesevelam hydrochloride group (Fig. 9-2), blood glucose level AUCs (0 to 120 min) in the colesevelam hydrochloride group (Fig. 9-3) in Example 3.
[Fig. 10] Drawings which depicts fasting blood glucose levels in the control group and the sevelamer hydrochloride group (Fig. 10-1), fasting insulin levels in the sevelamer hydrochloride group (Fig. 10-2) and blood glucose level AUCs (0 to 120 min) in the sevelamer hydrochloride group (Fig. 10-3) in Example 4.

### Best Mode for Carrying out the Invention

Hereafter, the present invention will be explained in more detail.

In the present invention, a pharmaceutically acceptable anion exchange resin means an anion exchange resin that can be administered as a medicament, and preferred examples thereof include anion exchange resins having a bile acid-adsorbing ability. The anion exchange resins are not particularly limited so long as they exhibit an insulin resistance-improving action when administered to hyperlipemia pathologic models such as explained in the examples mentioned later.

An example includes colestimide (2-methylimidazole-epichlorohydrin copolymers) which is the most preferred example. Although colestimide has an irregular and disordered complicated three-dimensional structure, the resin is represented by the basic structure of the following formula (I), of which structure is partially represented by the following formula (II), and can be obtained by a polymerization reaction of an epichlorohydrin derivative and an amine of which typical example is an imidazole derivatives, specifically, by the preparation method described in Japanese Patent Unexamined Publication (Kokai) No. 60-209523.

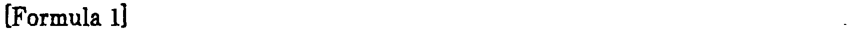

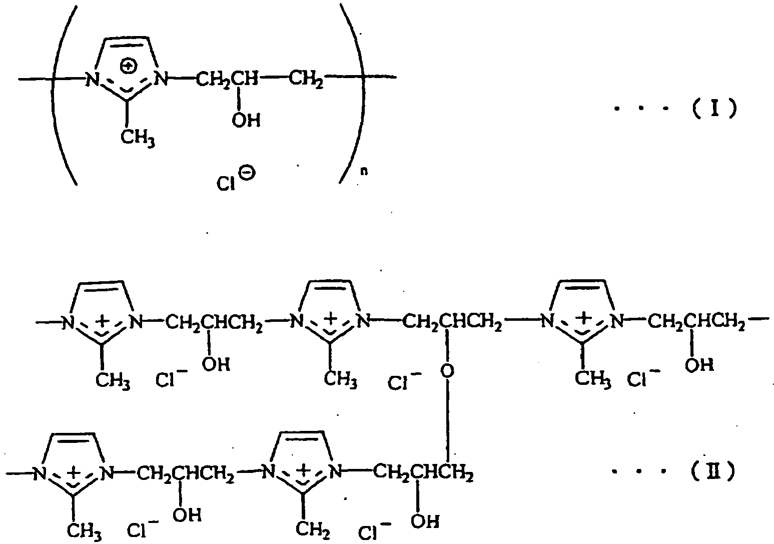

Colestimide is registered with a nonproprietary name of colestimide (chemical name: 2-methylimidazole-epichlorohydrin copolymer) in JAN. The resin is registered with a nonproprietary name of colestilan (chemical name: 2-methylimidazole polymer with 1-chloro-2,3-epoxypropane) in INN.

Other preferred examples of the anion exchange resin include the aforementioned cholestyramine resin, colestipol (N-(2-aminoethyl-N'-[2-[(2-aminoethyl)amino]ethyl]-1,2-ethanediamine polymer added with (chloromethyl)oxylane) and the like. These resins are sold by Sigma. The cholestyramine resin is a strong basic anion exchange resin containing a styrene-divinylbenzene copolymer added with quaternary ammonium groups, and the basic structure thereof is represented by the following formula (III).

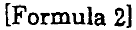

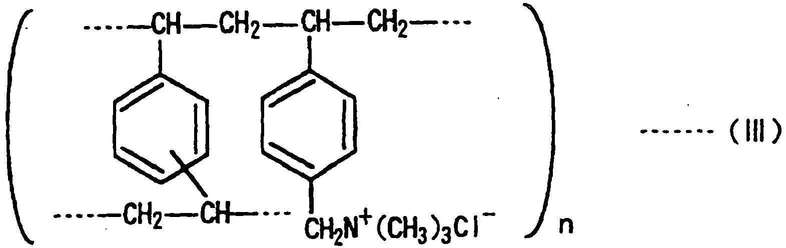

Further, the basic structure of sevelamer hydrochloride is represented by the following formula, and said resin can be prepared by the method described in U.S. Patent No. 5,496,545 or similar methods.

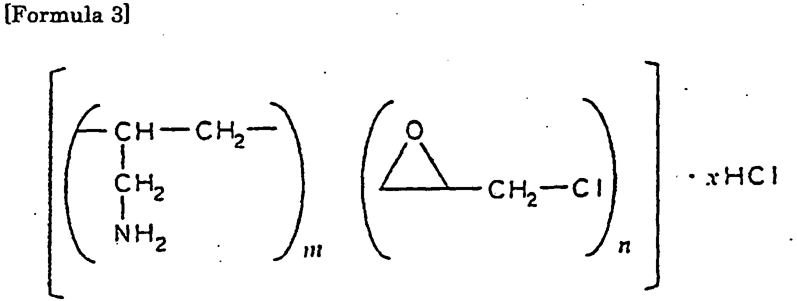

The basic structure of colesevelam hydrochloride is represented by the following formula. The resin can be prepared by the method described in U.S. Patent No. 5,607,669 or similar methods.

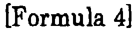

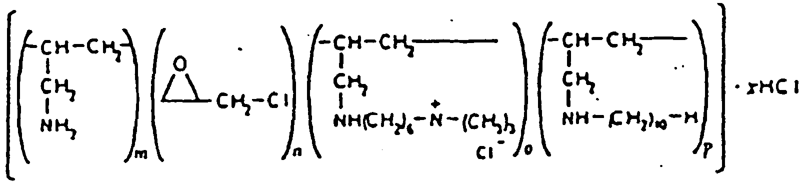

In addition, the anion exchange resins described in International Patent Unexamined Publication in Japanese (Kohyo) Nos. 9-504782, 9-500368, 10-501264, 10-501842, 11-507093, 11-512074, and 5-512332 and Japanese Patent Unexamined Publication (Kokai) Nos. 8-208750, 9-202732, 10-114661, and 11-228449 can also be used in the present invention so long as they do not depart from the gist of the present invention.

As the insulin resistance-improving agent of the present invention, the aforementioned compounds as an active ingredient per se may be used. Pharmaceutical compositions containing the aforementioned active ingredients and commonly used additives for pharmaceutical preparations are preferably prepared and used.

Examples of such pharmaceutical compositions include tablets, capsules, subtilized granules, pills, troches, solutions and the like, and these are orally administered (including sublingual administration).

Oral pharmaceutical compositions can be prepared by conventional methods such as mixing, filling and compressing. Further, the active ingredient may be distributed in a pharmaceutical composition by using a large amount of excipient and by applying repeated mixing operations. For example, tablets or capsules used for oral administration are preferably provided as unit administration products and may contain carriers ordinarily used for pharmaceutical preparations such as binders, fillers, diluents, compressing agents, lubricants, disintegrating agents, coloring materials, flavors, and wetting agents. Tablets may be prepared as, for example, coated tablets by using a coating agent according to methods known to those skilled in the art.

Preferred examples of the excipients include cellulose, mannitol, lactose and the like. Starch, polyvinylpyrrolidone, starch derivatives such as sodium starch glycolate and the like as disintegrating agents, sodium laurylsulfate as lubricant, and the like can be used as additives for pharmaceutical preparations. Examples of pharmaceutical compositions in the form of oral liquid include pharmaceutical compositions such as aqueous or oily suspensions, solutions, emulsions, syrups and elixirs and dry pharmaceutical compositions that can be redissolved in water or a suitable medium before use.

The above solutions may be mixed with ordinary additives, for example, suspending agents such as sorbitol, syrup, methylcellulose, gelatin, hydroxyethylcellulose, carboxymethylcellulose, aluminum stearate gel and hydrogenated edible fat; emulsifiers such as lecithin, sorbitan monooleate and gum arabic; oily esters such as almond oil, rectified coconut oil and glycerin esters; nonaqueous media such as propylene glycol and ethyl alcohol (edible oil may be included); preservatives such as methyl ester, ethyl ester or propyl ester of p-hydroxybenzoic acid and sorbic acid; usual flavors or coloring materials and the like, if necessary.

Where the aforementioned oral pharmaceutical composition is in the form of, for example, a tablet, capsule, subtilized granule or the like, the composition usually contains 5 to 95% by weight, preferably 25 to 90% by weight, of the active ingredient.

Colestimide is sold by Mitsubishi Pharma Corporation with a trade name of Cholebine. Cholebine, per se, may be used according to the present invention. Further, sevelamer hydrochloride is sold by Chugai Pharmaceutical Co., Ltd. and Genzyme Corporation with a trade name of Renagel and by Kirin Brewery Co., Ltd. with a trade name of Phosblock. Renagel, per se, may be used according to the present invention. Further, colesevelam hydrochloride is marketed by Sankyo Pharma Inc. with a trade name of WelChol. WelChol, per se, may be used according to the present invention.

A dose of the insulin resistance-improving agent of the present invention can be suitably selected depending on the active ingredient to be used, the age, health condition and body weight of a patient, severity of diseases, type and frequency of treatment simultaneously applied, nature of desired effect, and the like. In general, as for colestimide as an example, a daily dose of 1 to 60 g for an adult in terms of the amount of the active ingredient can be administered once or several times per day.

According to the present invention, the aforementioned pharmaceutically acceptable anion exchange resin and another oral hypoglycemic agent can be used simultaneously, separately, or successively. Specifically, a medicament comprising the aforementioned pharmaceutically acceptable anion exchange resin as an active ingredient and another oral hypoglycemic agent can be administered as a single pharmaceutical composition or as separate pharmaceutical compositions prepared on the basis of doses determined by suitable increase or decrease depending on the age, condition, sexuality, symptoms of a patient and the like. Where separate pharmaceutical compositions are administered, they can be administered in the same or different dosage forms simultaneously, in the same or different dosage forms at different times on the same day, or with given intervals over several days, several weeks or several months depending on the age, condition, sexuality, symptoms of a patient and the like.

Examples of the oral hypoglycemic agent used in the present invention include α-glucosidase inhibitors, biguanides, insulin sensitivity improving agents, sulfonylurea agents, rapid-acting insulin secretagogues and the like, but not limited to these examples. The α-glucosidase inhibitors are not particularly limited, and examples thereof include acarbose, voglibose and the like. The biguanides are not particularly limited, and examples thereof include metformin and the like. The insulin sensitivity improving agents are not particularly limited, and examples thereof include pioglitazone and the like. The sulfonylurea agents are not particularly limited, and examples thereof include gliclazide, glibenclamide, glimepide and the like. The insulin secretagogues are not particularly limited, and examples thereof include nateglinide and the like. As these oral hypoglycemic agents, those soled as reagents may be used or those that have already been launched as medicaments may be used.

In the above descriptions, embodiments as an insulin resistance-improving agent are mainly explained. However, the agent of the present invention can be used in the same manner for embodiments as an onset-suppressing and/or therapeutic agent for insulin resistance syndromes and prophylactic, improving and/or therapeutic agent for diseases and symptoms resulting from insulin resistance.

Insulin resistance syndrome was advocated by De Frozo in 1991 and is thought to be one of the onset mechanisms of diabetes. The resistance results in a risk of hyperlipemia and hypertension as well as diabetes. Obesity is considered to be a cause of the resistance. The resistance is also considered to be a direct cause of arteriosclerosis.

Specific examples of the diseases and symptoms resulting from insulin resistance include hyperinsulinism, fatty liver.

### Examples

The present invention will be explained more specifically with reference to the following examples. However, the scope of the present invention is not limited to these examples. The colestimide used in the following examples was prepared according to the method described in Japanese Patent Unexamined Publication (Kokai) No. 60-209523. Further, sevelamer hydrochloride and colesevelam hydrochloride used were appropriately prepared from sold products.

### Example 1

### 1. Test method

ApoE3 Leiden mice (male, TNO Pharma, Leiden, Netherlands) (n = 45) were fed with a high fat diet (45.4% fat) for 3 weeks and divided into two groups based on body weight and serum parameters (total cholesterol (TC), triglyceride level (TG) and glucose level (Glc)). One group (n = 30) was continuously fed with the high fat diet (high fat diet group), and the other group (n = 15) with the high fat diet containing 1.5% (w/w) colestimide (colestimide prophylactic treatment group).

Twelve weeks later, the high fat diet group was further divided into two groups based on body weight and the aforementioned serum parameters (TC, TG, Glc). One group was fed with the high fat diet (control group, n = 15), and the other group with the high fat diet containing 1.5% (w/w) colestimide (colestimide therapeutic treatment group, n = 15).

Eight weeks later, insulin resistance was measured by the hyperinsulinemic clamp method. An indwelling needle was inserted into the caudal vein of each animal under anesthesia (0.5 ml/kg Hypnorm and 12.5 mg/g midazalom), and insulin was infused to achieve a high insulin level (hyperinsulinemic state). To maintain the blood glucose level lowered by the insulin infusion at 7.0 mM, a 12.5% D-glucose solution was continuously infused with controlling the infusion rate. The insulin sensitivity index was represented in terms of infusion amount of the 12.5% D-glucose solution (glucose infusion rate) required to maintain the blood glucose level at 7.0 mM. Better insulin sensitivity increases infusion amount of D-glucose. Insulin sensitivity index = glucose infusion rate (mmol glucose/min/kg)

### 2. Results

### (1) Body weight

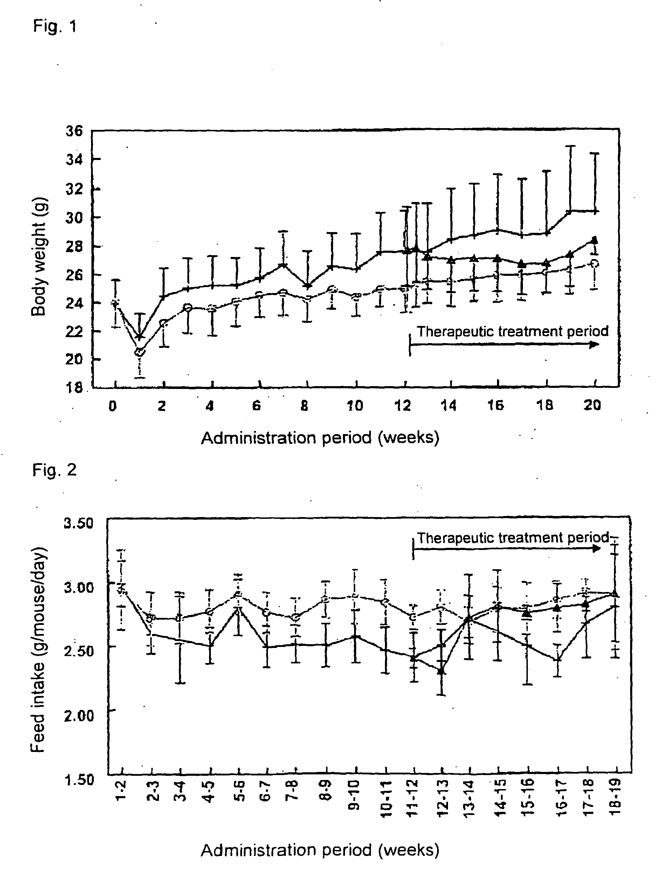

Changes in body weight in the control group (+), the colestimide prophylactic treatment group (o) and the colestimide therapeutic treatment group (A) are shown in Fig. 1. Body weights in the colestimide prophylactic treatment group (20-week treatment) were significantly lower than those in the control group (p < 0.05). Further, body weights in the colestimide therapeutic treatment group (8-week treatment from the 12th week) were not significantly different from, but always lower than, those in the control group.

### (2) Feed intake

Changes in feed intake in the control group (+), the colestimide prophylactic treatment group (o) and the colestimide therapeutic treatment group (A) are shown in Fig. 2. The feed intakes in the colestimide prophylactic treatment group were always higher from the 1st week of the treatment than those in the control group (p < 0.05). It can be understood that the feed intakes in the colestimide therapeutic treatment group were not significantly different from those in the control group, but more increased within a short period of time after the treatment with colestimide as compared with the control group.

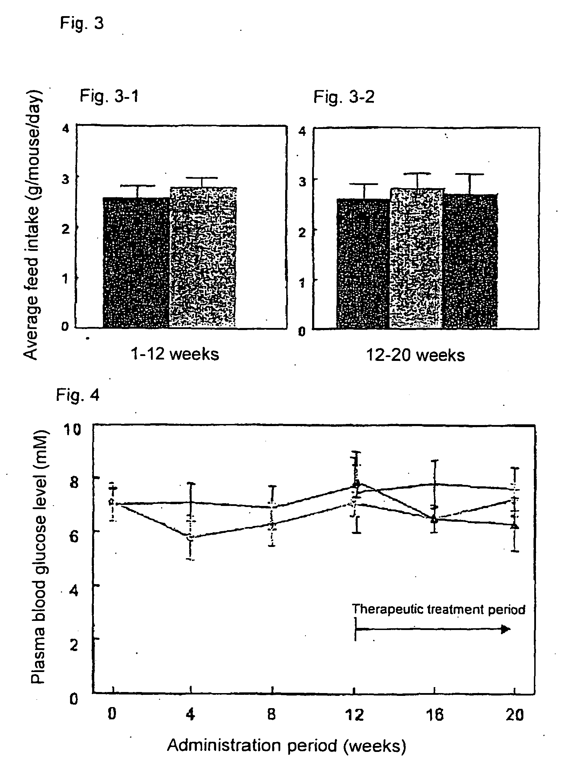

Comparison of the average feed intake during the 1st to 12th weeks of the test period is shown in Fig. 3-1, and comparison of the average feed intake during the 12th to 20th weeks of the test period is shown in Fig. 3-2. In the bar graph of Fig. 3-1, the left bar represents the result for the control group, and the right bar for the colestimide prophylactic treatment group. In the bar graph of Fig. 3-2, the left bar represents the result for the control group, the middle bar for the colestimide prophylactic treatment group, and the right bar for the colestimide therapeutic treatment group. The average feed intakes in the colestimide prophylactic treatment group and the colestimide therapeutic treatment group significantly increased as compared with those in the control group (p < 0.05).

From the results shown in Figs. 3-1, 3-2 and 1, it was revealed that lower body weights were observed in the colestimide prophylactic treatment group as compared with the control group in spite of the increase in feed intake.

### (3) Plasma sugar levels (glucose levels)

Changes in the plasma glucose level in the control group (+), the colestimide prophylactic treatment group (o) and the colestimide therapeutic treatment group (A) are shown in Fig. 4. The blood glucose levels significantly decreased in the colestimide prophylactic treatment group from the 2nd to 12th weeks, and in the colestimide prophylactic treatment group and the colestimide therapeutic treatment group in the 16th week as compared with those in the control group (p < 0.05).

### (4) Plasma insulin levels

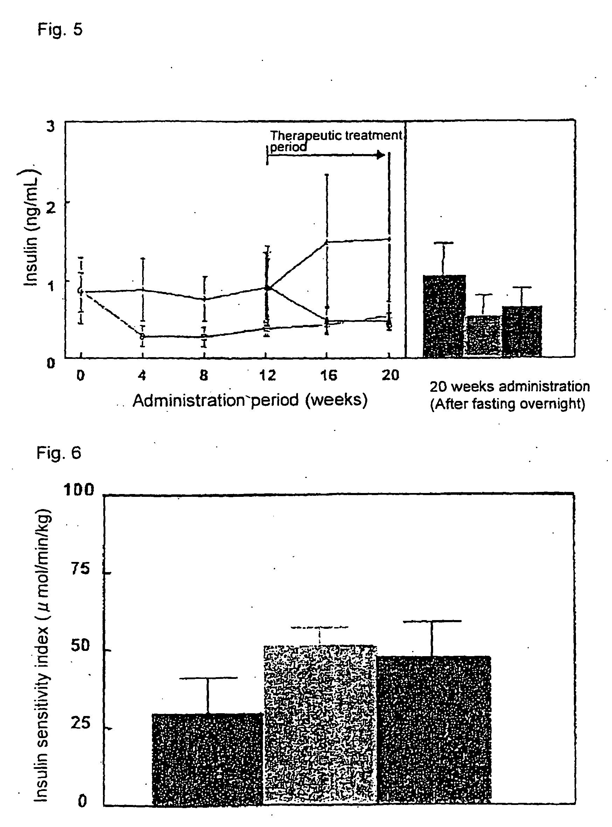

Changes in the plasma insulin level in the control group (+), the colestimide prophylactic treatment group (o) and the colestimide therapeutic treatment group (A) are shown in Fig. 5. In the bar graph of Fig. 5, the left bar represents the result for the control group, the middle bar for the colestimide prophylactic treatment group, and the right bar for the colestimide therapeutic treatment group. The insulin levels in the control group markedly increased after 16 weeks of the high fat diet loading. This result suggests that the mice were insulin resistant at that time. The insulin levels significantly decreased in both of the colestimide prophylactic treatment group and the colestimide therapeutic treatment group (p < 0.05).

### (5) Insulin sensitivity indices (insulin resistance-improving action)

The results are shown in Fig. 6. In the bar graph of Fig. 6, the left bar represents the result for the control group, the middle bar for the colestimide prophylactic treatment group, and the right bar for the colestimide therapeutic treatment group. The insulin sensitivity indices significantly increased in both of the colestimide prophylactic treatment group and the colestimide therapeutic treatment group as compared with those in the control group (p < 0.05), indicating improvement of insulin sensitivity.

### (6) Glucose uptake into tissues associated with insulin resistance improvement

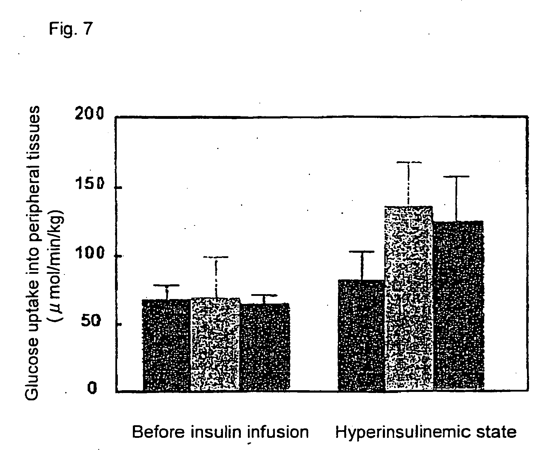

The results are shown in Fig. 7. In the bar graph of Fig. 7, the left bar represents the result for the control group, the middle bar for the colestimide prophylactic treatment group, and the right bar for the colestimide therapeutic treatment group. No significant difference was observed for all of the groups in glucose uptake into tissues under the condition before insulin infusion (basal), whist glucose uptakes into the peripheral tissues (skeletal muscles) in the hyperinsulinemic state significantly increased in the colestimide prophylactic treatment group and the colestimide therapeutic treatment group as compared with those in the control group (p < 0.05). This result indicates that glucose uptake into the peripheral tissues increased only in the presence of insulin, and thus improvement of insulin sensitivity was demonstrated.

From the above results, it is clearly understood that colestimide exhibits an insulin resistance-improving action, and this action is not resulting from suppression of feed intake or glucose absorption from the gastrointestinal tract.

### Example 2

### 1. Test method

KKAy mice (male, Clea Japan, Inc., n = 8) were used. The control group was fed with a high fat diet (23.6% fat), and the colestimide group was fed with the high fat diet containing 2% colestimide. A glucose tolerance test was performed according to a conventional method after 2 weeks of the treatment. The mice were fasted overnight, and blood was collected before loading of glucose. Then, a glucose solution was orally given, and blood glucose levels were measured after 30, 60, 90 and 120 minutes. The blood glucose level AUCs (0 to 120 min) were calculated by using the blood glucose levels obtained. The fasting blood glucose levels and the fasting insulin levels were measured by using the blood samples collected before the loading of glucose.

### 2. Results

### (1) Fasting blood glucose levels

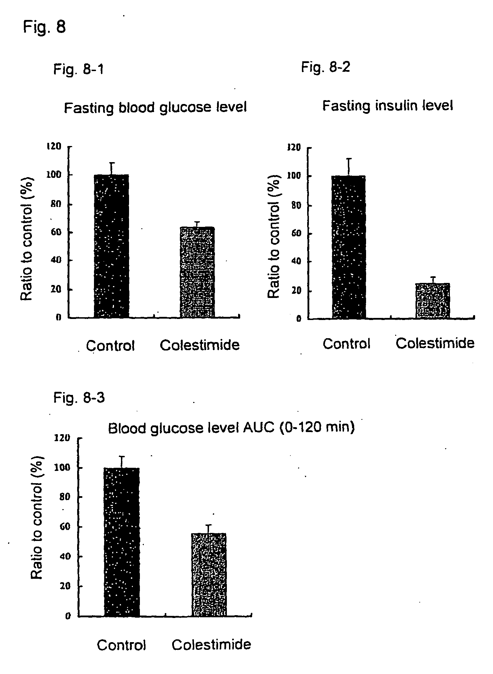

Fasting blood glucose levels in the control group and the colestimide group are shown in Fig. 8-1. The fasting blood glucose levels in the colestimide group significantly decreased as compared with those in the control group (p < 0.01).

### (2) Fasting insulin levels

Fasting insulin levels in the control group and the colestimide group are shown in Fig. 8-2. The fasting insulin levels in the colestimide group significantly decreased as compared with those in the control group (p < 0.01).

### (3) Blood glucose level AUCs (0 to 120 min)

Blood glucose level AUCs (0 to 120 min) in the control group and the colestimide group are shown in Fig. 8-3. The blood glucose level AUCs (0 to 120 min) in the colestimide group significantly decreased as compared with those in the control group (p < 0.01).

The above results suggested that colestimide had an insulin resistance improving action.

### Example 3

### 1. Test method

KKAy mice (male, Clea Japan, Inc., n = 8) were used. The control group was fed with a high fat diet (23.6% fat), and the colesevelam hydrochloride group was fed with the high fat diet containing 2% colesevelam hydrochloride. A glucose tolerance test was performed according to a conventional method after 2 weeks of the treatment. The mice were fasted overnight, and blood was collected before loading of glucose. Then, a glucose solution was orally given, and blood glucose levels were measured after 30, 60, 90 and 120 minutes. The blood glucose level AUCs (0 to 120 min) were calculated by using the blood glucose levels obtained. The fasting blood glucose levels and the fasting insulin levels were measured by using the blood samples collected before the loading of glucose.

### 2. Results

### (1) Fasting blood glucose levels

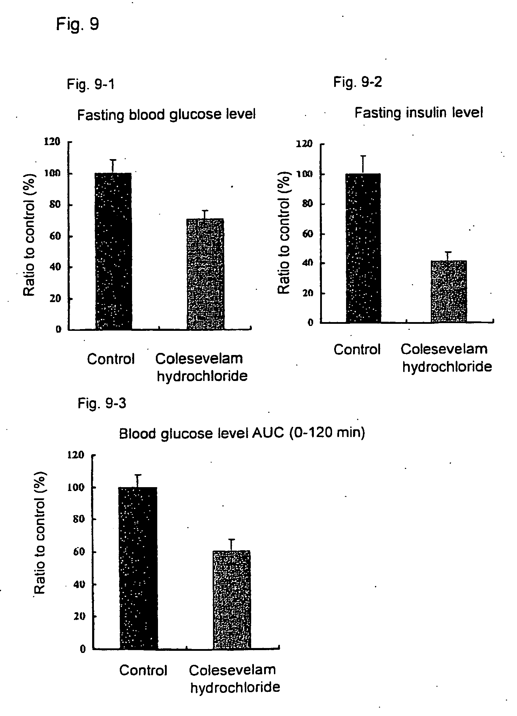

Fasting blood glucose levels in the control group and the colesevelam hydrochloride group are shown in Fig. 9-1. The fasting blood glucose levels in the colesevelam hydrochloride group significantly decreased as compared with those in the control group (p < 0.01).

### (2) Fasting insulin levels

Fasting insulin levels in the control group and the colesevelam hydrochloride group are shown in Fig. 9-2. The fasting insulin levels in the colesevelam hydrochloride group significantly decreased as compared with those in the control group (p < 0.01).

### (3) Blood glucose level AUCs (0 to 120 min)

Blood glucose level AUCs (0 to 120 min) in the control group and the colesevelam hydrochloride group are shown in Fig. 9-3. The blood glucose level AUCs (0 to 120 min) in the colesevelam hydrochloride group significantly decreased as compared with those in the control group (p < 0.01).
The above results suggested that colesevelam hydrochloride had an insulin resistance-improving action.

### Example 4

### 1. Test method

KKAy mice (male, Clea Japan, Inc., n = 8) were used. The control group was fed with a high fat diet (23.6% fat), and the sevelamer hydrochloride group was fed with the high fat diet containing 2% sevelamer hydrochloride. A glucose tolerance test was performed according to a conventional method after 2 weeks of the treatment. The mice were fasted overnight, and blood was collected before loading of glucose. Then, a glucose solution was orally given, and blood glucose levels were measured after 30, 60, 90 and 120 minutes. The blood glucose level AUCs (0 to 120 min) were calculated by using the obtained blood glucose levels. The fasting blood glucose levels and the fasting insulin levels were measured by using the blood samples collected before the loading of glucose.

### 2. Results

### (1) Fasting blood glucose levels

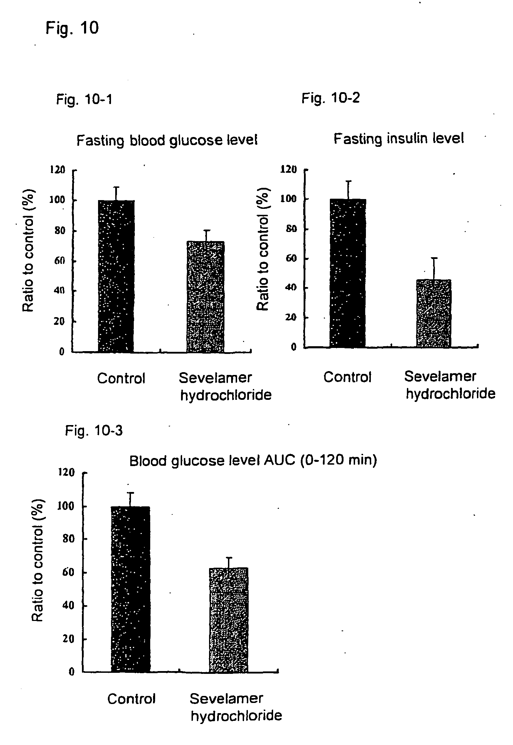

Fasting blood glucose levels in the control group and the sevelamer hydrochloride group are shown in Fig. 10-1. The fasting blood glucose levels in the colesevelam hydrochloride group significantly decreased as compared with those in the control group (p < 0.05).

### (2) Fasting insulin levels

Fasting insulin levels in the control group and the sevelamer hydrochloride group are shown in Fig. 10-2. The fasting insulin levels in the sevelamer hydrochloride group significantly decreased as compared with those in the control group (p < 0.01).

### (3) Blood glucose level AUCs (0-120 min)

Blood glucose level AUCs (0 to 120 min) in the control group and the sevelamer hydrochloride group are shown in Fig. 10-3. The blood glucose level AUCs (0 to 120 min) in the sevelamer hydrochloride group significantly decreased as compared with those in the control group (p < 0.01).

The above results suggested that sevelamer hydrochloride had an insulin resistance-improving action.

### Industrial Applicability

According to the present invention, a medicament can be provided which exhibits an insulin resistance-improving action regardless of volume of meal or glucose absorption from the gastrointestinal tract.

## Claims

1. An agent comprising a pharmaceutically acceptable anion exchange resin as an active ingredient for use in the improvement of insulin resistance.

2. The agent according to claim 1, wherein the pharmaceutically acceptable anion exchange resin has a bile acid-adsorbing ability.

3. The agent according to claim 1 or 2, wherein the pharmaceutically acceptable anion exchange resin is selected from the group consisting of colestimide, cholestyramine resin, colestipol, sevelamer hydrochloride, and colesevelam hydrochloride.

4. The agent according to claim 1 or 2, wherein the pharmaceutically acceptable anion exchange resin is an anion exchange resin synthesized by a polymerization reaction of an epichlorohydrin derivative and an amine of which typical example includes an imidazole derivative.

5. The agent according to any one of claims 1 to 4, wherein the pharmaceutically acceptable anion exchange resin is colestimide.

6. The agent according to any one of claims 1 to 5, with which an oral hypoglycemic agent is used simultaneously, separately, or successively.

7. The agent according to claim 6, wherein the oral hypoglycemic agent is selected from the group consisting of α-glucosidase inhibitors, biguanides, insulin sensitivity improving agents, sulfonylurea agents, rapid-acting insulin secretagogues, pharmaceutical preparations comprising GLP-1 or derivatives thereof, and DPP-IV inhibitors.

8. An agent for use in the prophylactic, improving and/or therapeutic treatment of a disease or symptom resulting from insulin resistance, which comprises a pharmaceutically acceptable anion exchange resin as an active ingredient, wherein the disease or symptom resulting from insulin resistance is selected from the group consisting of hyperinsulinism and fatty liver.

9. The agent according to claim 8, wherein the disease or symptom resulting from insulin resistance is hyperinsulinism.

10. The agent according to claim 8, wherein the disease or symptom resulting from insulin resistance is fatty liver.

11. The agent according to any one of claims 8 to 10, wherein the agent is one for prophylactic treatment of the disease or symptom resulting from insulin resistance.

12. The agent according to any one of claims 8 to 10, wherein the agent is one for improving treatment of the disease or symptom resulting from insulin resistance.

13. The agent according to any one of claims 8 to 10, wherein the agent is one for therapeutic treatment of the disease or symptom resulting from insulin resistance.

14. The agent according to any one of claims 8 to 13, wherein the pharmaceutically acceptable anion exchange resin has a bile acid adsorbing ability.

15. The agent according to any one of claims 8 to 14, wherein the pharmaceutically acceptable anion exchange resin is selected from the group consisting of colestimide, cholestyramine resin, colestipol, sevelamer hydrochloride, and colesevelam hydrochloride.

16. The agent according to any one of claims 8 to 15, wherein the pharmaceutically acceptable anion exchange resin is an anion exchange resin synthesized by a polymerization reaction of an epichlorohydrin derivative and an amine of which typical example includes an imidazole derivative.

17. The agent according to any one of claims 8 to 16, wherein the pharmaceutically acceptable anion exchange resin is colestimide.

18. The agent according to any one of claims 8 to 17, with which an oral hypoglycemic agent is used simultaneously, separately, or successively.

19. The agent according to claim 18, wherein the oral hypoglycemic agent is selected from the group consisting of α-glucosidase inhibitors, biguanides, insulin sensitivity improving agents, sulfonylurea agents, rapid-acting insulin secretagogues, pharmaceutical preparations comprising GLP-1 or derivatives thereof, and DPP-IV inhibitors.

## Patentansprüche

1. Mittel, umfassend ein pharmazeutisch annehmbares Anionenaustauschharz als Wirkstoff zur Verwendung bei der Verbesserung der Insulinresistenz.

2. Mittel nach Anspruch 1, wobei das pharmazeutisch annehmbare Anionenaustauschharz die Fähigkeit hat, Gallensäure zu adsorbieren.

3. Mittel nach Anspruch 1 oder 2, wobei das pharmazeutisch annehmbare Anionenaustauschharz aus der Gruppe gewählt ist, bestehend aus Colestimid, Cholestyraminharz, Colestipol, Sevelamerhydrochlorid, und Colesevelamhydrochlorid.

4. Mittel nach Anspruch 1 oder 2, wobei das pharmazeutisch annehmbare Anionenaustauschharz ein Anionenaustauschharz ist, synthetisiert durch eine Polymerisationsreaktion eines Epichlorhydrinderivates und eines Amins, wobei ein typisches Beispiel hierfür ein Imidazolderivat beinhaltet.

5. Mittel nach mindestens einem der Ansprüche 1 bis 4, wobei das pharmazeutisch annehmbare Anionenaustauschharz Colestimid ist.

6. Mittel nach mindestens einem der Ansprüche 1 bis 5, mit dem ein orales hypoglykämisches Mittel gleichzeitig, separat oder aufeinanderfolgend angewendet wird.

7. Mittel nach Anspruch 6, wobei das orale hypoglykämische Mittel aus der Gruppe gewählt ist, bestehend aus α-Glucosidaseinhibitoren, Biguaniden, die Insulinempfindlichkeit verbessernden Mitteln, Sulfonylharnstoffmitteln, schnell wirkenden Insulinsekretions-anregenden Mitteln, pharmazeutischen Präparaten, umfassend GLP-1 oder Derivate hiervon, und DPP-IV-Inhibitoren.

8. Mittel zur Verwendung bei der prophylaktischen, verbessernden und/oder therapeutischen Behandlung einer Krankheit oder eines Symptoms, resultierend aus Insulinresistenz, welches ein pharmazeutisch annehmbares Anionenaustauschharz als Wirkstoff umfasst, wobei die Krankheit oder das Symptom, resultierend aus Insulinresistenz, gewählt ist aus der Gruppe, bestehend aus Hyperinsulinismus und Fettleber.

9. Mittel nach Anspruch 8, wobei die Krankheit oder das Symptom, resultierend aus Insulinresistenz, Hyperinsulinismus ist.

10. Mittel nach Anspruch 8, wobei die Krankheit oder das Symptom, resultierend aus Insulinresistenz, Fettleber ist.

11. Mittel nach mindestens einem der Ansprüche 8 bis 10, wobei das Mittel eines für die prophylaktische Behandlung der Krankheit oder des Symptoms, resultierend aus Insulinresistenz, ist.

12. Mittel nach mindestens einem der Ansprüche 8 bis 10, wobei das Mittel eines für die verbessernde Behandlung der Krankheit oder des Symptoms, resultierend aus Insulinresistenz, ist.

13. Mittel nach mindestens einem der Ansprüche 8 bis 10, wobei das Mittel eines für die therapeutische Behandlung der Krankheit oder des Symptoms, resultierend aus Insulinresistenz, ist.

14. Mittel nach mindestens einem der Ansprüche 8 bis 13, wobei das pharmazeutisch annehmbare Anionenaustauschharz die Fähigkeit hat, Gallensäure zu adsorbieren.

15. Mittel nach mindestens einem der Ansprüche 8 bis 14, wobei das pharmazeutisch annehmbare Anionenaustauschharz aus der Gruppe gewählt ist, bestehend aus Colestimid, Cholestyraminharz, Colestipol, Sevelamerhydrochlorid, und Colesevelamhydrochlorid.

16. Mittel nach mindestens einem der Ansprüche 8 bis 15, wobei das pharmazeutisch annehmbare Anionenaustauschharz ein Anionenaustauschharz ist, synthetisiert durch eine Polymerisationsreaktion eines Epichlorhydrinderivates und eines Amins, wobei ein typisches Beispiel hierfür ein Imidazolderivat beinhaltet.

17. Mittel nach mindestens einem der Ansprüche 8 bis 16, wobei das pharmazeutisch annehmbare Anionenaustauschharz Colestimid ist.

18. Mittel nach mindestens einem der Ansprüche 8 bis 17, mit dem ein orales hypoglykämisches Mittel gleichzeitig, separat oder aufeinanderfolgend angewendet wird.

19. Mittel nach Anspruch 18, wobei das orale hypoglykämische Mittel aus der Gruppe gewählt ist, bestehend aus α-Glucosidaseinhibitoren, Biguaniden, die Insulinempfindlichkeit verbessernden Mitteln, Sulfonylharnstoffmitteln, schnell wirkenden Insulinsekretions-anregenden Mitteln, pharmazeutischen Präparaten, umfassend GLP-1 oder Derivate hiervon, und DPP-IV-Inhibitoren.

## Revendications

1. Agent comprenant une résine d'échange d'anions pharmaceutiquement acceptable en tant que principe actif pour une utilisation dans l'amélioration de l'insulinorésistance.

2. Agent selon la revendication 1, dans lequel la résine d'échange d'anions pharmaceutiquement acceptable a une capacité d'adsorption d'acide biliaire.

3. Agent selon la revendication 1 ou 2, dans lequel la résine d'échange d'anions pharmaceutiquement acceptable est choisie dans le groupe constitué par le colestimide, la résine cholestyramine, le colestipol, le chlorhydrate de sevelamer, et le chlorhydrate de colésévélam.

4. Agent selon la revendication 1 ou 2, dans lequel la résine d'échange d'anions pharmaceutiquement acceptable est une résine d'échange d'anions synthétisée par une réaction de polymérisation d'un dérivé d'épichlorhydrine et d'une amine dont un exemple typique comprend un dérivé d'imidazole.

5. Agent selon l'une quelconque des revendications 1 à 4, dans lequel la résine d'échange d'anions pharmaceutiquement acceptable est le colestimide.

6. Agent selon l'une quelconque des revendications 1 à 5, avec lequel un agent hypoglycémique oral est utilisé simultanément, séparément ou successivement.

7. Agent selon la revendication 6, dans lequel l'agent hypoglycémique oral est choisi dans le groupe constitué par les inhibiteurs de l'α-glucosidase, les biguanides, les agents améliorant la sensibilité à l'insuline, les agents de sulfonylurée, les sécrétagogues d'insuline à action rapide, les préparations pharmaceutiques comprenant le GLP-1 ou des dérivés de celui-ci, et les inhibiteurs de la DPP-IV.

8. Agent pour une utilisation dans le traitement prophylactique, d'amélioration et/ou thérapeutique d'une maladie ou d'un symptôme dû à une insulinorésistance, qui comprend une résine d'échange d'anions pharmaceutiquement acceptable comme principe actif, dans lequel la maladie ou le symptôme dû à une insulinorésistance est choisi dans le groupe constitué par l'hyperinsulinisme et la stéatose hépatique.

9. Agent selon la revendication 8, dans lequel la maladie ou le symptôme dû à une insulinorésistance est l'hyperinsulinisme.

10. Agent selon la revendication 8, dans lequel la maladie ou le symptôme dû à une insulinorésistance est la stéatose hépatique.

11. Agent selon l'une quelconque des revendications 8 à 10, l'agent étant destiné à un traitement prophylactique de la maladie ou du symptôme dû à une insulinorésistance.

12. Agent selon l'une quelconque des revendications 8 à 10, l'agent étant destiné à un traitement d'amélioration de la maladie ou du symptôme dû à une insulinorésistance.

13. Agent selon l'une quelconque des revendications 8 à 10, l'agent étant destiné à un traitement thérapeutique de la maladie ou du symptôme dû à une insulinorésistance.

14. Agent selon l'une quelconque des revendications 8 à 13, dans lequel la résine d'échange d'anions pharmaceutiquement acceptable a une capacité d'adsorption d'acide biliaire.

15. Agent selon l'une quelconque des revendications 8 à 14, dans lequel la résine d'échange d'anions pharmaceutiquement acceptable est choisie dans le groupe constitué par le colestimide, la résine cholestyramine, le colestipol, le chlorhydrate de sevelamer, et le chlorhydrate de colésévélam.

16. Agent selon l'une quelconque des revendications 8 à 15, dans lequel la résine d'échange d'anions pharmaceutiquement acceptable est une résine d'échange d'anions synthétisée par une réaction de polymérisation d'un dérivé d'épichlorhydrine et d'une amine dont un exemple typique comprend un dérivé d'imidazole.

17. Agent selon l'une quelconque des revendications 8 à 16, dans lequel la résine d'échange d'anions pharmaceutiquement acceptable est le colestimide.

18. Agent selon l'une quelconque des revendications 8 à 17, avec lequel un agent hypoglycémique oral est utilisé simultanément, séparément ou successivement.

19. Agent selon la revendication 18, dans lequel l'agent hypoglycémique oral est choisi dans le groupe constitué par les inhibiteurs de l'α-glucosidase, les biguanides, les agents améliorant la sensibilité à l'insuline, les agents de sulfonylurée, les sécrétagogues d'insuline à action rapide, les préparations pharmaceutiques comprenant le GLP-1 ou des dérivés de celui-ci, et les inhibiteurs de la DPP-IV.
